# EUROPÄISCHE PATENTSCHRIFT

(11) **EP 1 363 937 B1**
(45) Veröffentlichungstag und Bekanntmachung des Hinweises auf die Patenterteilung: **05.11.2008**
(21) Anmeldenummer: 02727341.6
(22) Anmeldetag: 01.03.2002
(51) Int. Cl.: C07K 14/435, C12N 15/63, C07K 16/28, A61K 38/17, A61P 37/00, C07K 1/36, A61K 48/00

(54) **IMMUNMODULIERENDE WIRKSTOFFE AUS PARASITÄREN WÜRMERN UND VERFAHREN ZU DEREN ISOLIERUNG**
IMMUNOMODULATING AGENTS FROM PARASITIC WORMS AND METHOD FOR ISOLATION THEREOF
IMMUNOMODULANTS PROVENANT DE VERS PARASITES ET PROCEDE POUR LEUR SEPARATION

(30) Priorität: 01.03.2001 DE 10109844
(43) Veröffentlichungstag der Anmeldung: 26.11.2003
(73) Patentinhaber: Forschungszentrum Borstel Zentrum für Medizin und Biowissenschaften, 23845 Borstel (DE)
(72) Erfinder: SCHRAMM, Gabriele, 23867 Sülfeld (DE); HAAS, Helmut, 23867 Sülfeld (DE); FALCONE, Franco, Nottingham NG8 1NF (GB); GRONOW, Achim, 23867 Sülfeld (DE); HAISCH, Karin, University at Buffalo, Buffalo, NY 14214 (DE)
(74) Vertreter: UEXKÜLL & STOLBERG
(86) Internationale Anmeldenummer: PCT/EP2002/002228
(87) Internationale Veröffentlichungsnummer: WO 2002/085930

(56) Entgegenhaltungen:
- Schramm G. et al.:"The IL-4-inducing activity contained in Schistosoma mansoni egg antigen (SmEA) is a secretory glycoprotein" ALLERGY, 2001,vol 56,Suppl. 68,pg 109-10 XXTh Congress of the European Academy of allergology and clinical immunology. Berlin, Germany. May 09-13, 2001 XP008010124
- HAISCH K. ET AL.: "A glycoprotein from Schistosoma mansoni eggs ginds non-antigen-specific immunoglobulin E and releases interleukin-4 from human basophils" PARASITE IMMUNOLOGY, Bd. 23, August 2001 (2001-08), Seiten 427-434, XP002219794
- FALCONE F.H. ET AL.: "Human basophils release interleukin-4 after stimulation with Schistosoma mansoni egg antigen" EUROPEAN JOURNAL OF IMMUNOLOGY, Bd. 26, 1996, Seiten 1147-1155, XP008010115
- FINKELMAN F D ET AL: "The role of IL-13 in helminth-induced inflammation and protective immunity against nematode infections" CURRENT OPINION IN IMMUNOLOGY, CURRENT BIOLOGY LTD, XX, Bd. 11, Nr. 4, August 1999 (1999-08), Seiten 420-426, XP004257560 ISSN: 0952-7915
- DATABASE SWALL [Online] 1. Juni 1998 (1998-06-01) JAKUBCZAK J. ET AL.: "Hepatocyte growth factor NK1" Database accession no. O55027 XP002219796
- DATABASE EM_EST [Online] 12. Juli 1999 (1999-07-12) OLIVEIRA G.C. ET AL.: "ME000944.FOR Egg stage cDNA expression library in Lambda ZAPII Schistosoma mansoni cDNA 5', mRNA sequence" Database accession no. AI820476 XP002219797
- KING C.L. ET AL.: "Murine schistosomiasis mansoni: coordinate cytokine regulation and differences in cellular immune response of granuloma cells and splenocytes to endogenous and exogenous schistosome egg antigens" PARASITE IMMUNOLOGY, Bd. 23, November 2001 (2001-11), Seiten 607-615, XP002219795

### SEQUENZPROTOKOLL

<110> Forschungszentrum Borstel
<120> Immunmodulierende Wirkstoffe aus parasitaeren Wuermern und Verfahren zu deren Isolierung
<130> P059569
<140>
   <141>
<160> 2
<170> PatentIn Ver. 2.1
<210> 1
   <211> 405
   <212> DNA
   <213> Schistosoma mansoni
<220>
   <221> CDS
   <222> (1)..(405)
<400> 1
   atg ttt ctt att gcc gta ttg tca tac aca ttg ata agt caa ttg ggg      48
   Met Phe Leu Ile Ala Val Leu Ser Tyr Thr Leu Ile Ser Gln Leu Gly
     1               5                  10                  15
   ata act aca tcg gat tca tgc aaa tat tgt cta caa ttg tac gat gaa      96
   Ile Thr Thr Ser Asp Ser Cys Lys Tyr Cys Leu Gln Leu Tyr Asp Glu
                20                  25                  30
   acg tat gag agg ggt tca tat att gaa gtc tac aaa agc gtt ggc tca      144
   Thr Tyr Glu Arg Gly Ser Tyr Ile Glu Val Tyr Lys Ser Val Gly Ser
            35                  40                  45
   ctc tca cca cca tgg aca cct gga tct gtt tgt gta ccc ttc gta aat      192
   Leu Ser Pro Pro Trp Thr Pro Gly Ser Val Cys Val Pro Phe Val Asn
        50                  55                  60
   gac acg aag aga gag cgt cca tac tgg tat tta ttt gac aac gtc aat      240
   Asp Thr Lys Arg Glu Arg Pro Tyr Trp Tyr Leu Phe Asp Asn Val Asn
    65                  70                  75                  80
   tac aca ggt cgg att act ggt ctc gga cat ggt acc tgc att gat gac      288
   Tyr Thr Gly Arg Ile Thr Gly Leu Gly His Gly Thr Cys Ile Asp Asp
                    85                  90                  95
   ttc acg aaa tcc gga ttc aaa ggc att tcc tct att aaa cgg tgt att      336
   Phe Thr Lys Ser Gly Phe Lys Gly Ile Ser Ser Ile Lys Arg Cys Ile
               100                 105                 110
   caa aca aag gat gga aaa gtt gaa tgt atc aat caa ccg aaa cgg aga      384
   Gln Thr Lys Asp Gly Lys Val Glu Cys Ile Asn Gln Pro Lys Arg Arg
           115                 120                 125
   agg aca tac tgt cga ttc taa                                          405
   Arg Thr Tyr Cys Arg Phe
       130                 135
<210> 2
   <211> 134
   <212> PRT
   <213> Schistosoma mansoni
<400> 2
   Met Phe Leu Ile Ala Val Leu Ser Tyr Thr Leu Ile Ser Gln Leu Gly
    1               5                  10                  15
   Ile Thr Thr Ser Asp Ser Cys Lys Tyr Cys Leu Gln Leu Tyr Asp Glu
               20                  25                  30
   Thr Tyr Glu Arg Gly Ser Tyr Ile Glu Val Tyr Lys Ser Val Gly Ser
           35                  40                  45
   Leu Ser Pro Pro Trp Thr Pro Gly Ser Val Cys Val Pro Phe Val Asn
       50                  55                  60
   Asp Thr Lys Arg Glu Arg Pro Tyr Trp Tyr Leu Phe Asp Asn Val Asn
   65                  70                  75                  80
   Tyr Thr Gly Arg Ile Thr Gly Leu Gly His Gly Thr Cys Ile Asp Asp
                   85                  90                  95
   Phe Thr Lys Ser Gly Phe Lys Gly Ile Ser Ser Ile Lys Arg Cys Ile
               100                 105                 110
   Gln Thr Lys Asp Gly Lys Val Glu Cys Ile Asn Gln Pro Lys Arg Arg
           115                 120                 125
   Arg Thr Tyr Cys Arg Phe
       130

## Beschreibung

Die Erfindung betrifft Wirkstoffe aus parasitären Würmern, insbesondere *Schistosoma mansoni* und *Schistosoma japonicum*, die eine T-Helfer Typ 2 Antwort (Th2-Immunantwort) induzieren.

### Hintergrund der Erfindung:

Bei einer Reihe von Erkrankungen ist ursächlich eine pathologische Th1- oder eine abgeschwächte Th2-Reaktion nachgewiesen worden, bzw. es werden diese Reaktionen diskutiert. Tierexperimentelle Befunde lassen vermuten, dass bei diesen Erkrankungen eine Umstimmung der Immunantwort in Richtung Th2 protektiv wirkt. Zu nennen sind hier Organ-spezifische Autoimmun-Krankheiten wie Multiple Sklerose [Shevach, E.M. et al., Springer Semin. Immunopathol. 21 (1999) 249-262; Leonard, J.P. et al., Crit. Rev. Immunol. 17 (1997) 545-553], autoimmune Uveitis [Singh, V.K. et al., Immunol. Res. 20 (1999) 147-161; Sun, B. et al., Int. Immunol. 11 (1999) 1307-1312; Egwuagu, C.E. et al., J. Immunol. 162 (1999) 510-517], Insulin-pflichtiger Diabetes mellitus [Rabinovitch, A. et al., Biochem. Pharmacol. 55 (1998) 1139-1149; Cooke, A. et al., Parasite Immunol. 21 (1999) 169-176], rheumatoide Arthritis [Muller, B. et al., Springer Semin. Immunopathol. 20 (1998) 181-196], Behcet's Syndrom [Frassanito, M.A. et al., Arthritis Rheum. 42 (1999) 1967-1974] weiterhin die *Helicobacter pylori*-Infektion (die Ursache u.a. von Magenulcus und atrophischer Gastritis) [Smythies, L.E. et al., J. Immunol. 165 (2000) 1022-1029; Fox, J.G. et al., Nat. Med. 6 (2000) 536-542; Mattapallil, J.J. et al., Gastroenterology 118 (2000) 307-315], entzündliche Darmerkrankungen wie Morbus Crohn und andere [Romagnani, P. et al., Curr. Opin. Immunol. 9 (1997) 793-799; MacDonald, T.T., Curr. Top. Microbiol. Immunol. 236 (1999) 113-135], die akute Organtransplantat-Abstoßungsreaktion [Morelli, A.E. et al., Transplantation 69 (2000) 2647-2657] und spontane rekurrente Aborte [Jenkins, C. et al., Fertil. Steril. 73 (2000) 1206-1208].

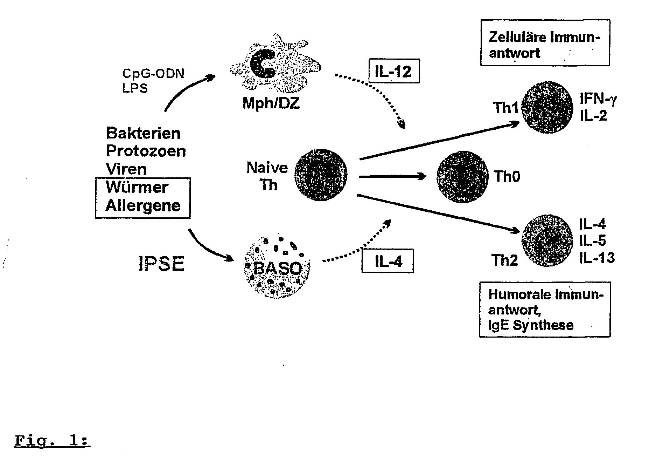

Für die Weichenstellung zur Th1- bzw. Th2-Antwort werden Zytokine als Schlüsselfaktoren angesehen [Paul, W.E. et al., Cell 76 (1994) 241-251]. Dabei dürfte IL-4 das entscheidende Zytokinsignal für die Differenzierung von naiven T-Helferzellen zu Th2-Zellen darstellen. Die Weichenstellung zur Th1-Antwort wird dagegen im wesentlichen durch IL-12 und IFN-γ kontrolliert, welche durch dendritische Zellen und andere akzessorische Zellen produziert werden. Abgesehen von Zytokinen können auch andere Faktoren die T-Zelldifferenzierung beeinflussen [Constant,S.L. et al., Annu.Rev.Immunol. 15 (1997) 297-322]. Für die Weichenstellung müssen IL-4 bzw. IL-12 bereits zum Zeitpunkt des *Priming* anwesend sein, d.h. früh während einer Immunantwort (vgl. Fig. 1).

Die frühe Produktion von IL-4 oder IL-12 und damit die T-ZellDifferenzierung werden durch exogene und endogene Faktoren kontrolliert. Unter den exogenen Faktoren ist vor allem die Art des Pathogens wichtig. Einige Pathogene stimulieren präferentiell eine Th1, andere eine Th2-Antwort [Scott,P. et al., Immunol. Today 12 (1991) 346-348]. Dies lässt vermuten, dass molekulare Eigenschaften des entsprechenden Pathogens verantwortlich für den jeweiligen Effekt sind. Da die Art des Pathogens die frühe Zytokinproduktion entscheidend beeinflusst, stellt sich die grundsätzliche Frage, welches die molekularen Triggerfaktoren sind, die frühes IL-4 bzw. frühes IL-12 induzieren, und welches die zelluläre Quelle ist, die diese Zytokine bereitstellt.

Die zelluläre Quelle für das frühe IL-4 ist Gegenstand einer kontroversen Diskussion [Coffman,R.L. et al., J. Exp. Med. 185 (1997) 373-375]. Grundsätzlich sind mehrere Zelltypen fähig, IL-4 zu produzieren, wenn sie entsprechend stimuliert werden: Typ 2-T-Zellen, murine NK1.1⁺ T-Zellen, Eosinophile, Mastzellen und Basophile. Während einer primären Immunantwort dürfte das frühe IL-4 sehr wahrscheinlich von Zellen der angeborenen Immunität stammen, da zum Zeitpunkt des Erstkontaktes mit einem Immunogen reife Antigen-spezifische T-Zellen noch nicht zur Verfügung stehen. Selbst im Falle präexistenter kreuzreaktiver Th2-Zellen bleibt die Frage, welche Struktur deren Entwicklung ausgelöst hat. Damit sind Typ 2-T-Zellen als Quelle des frühen IL-4 auszuschließen. Die postulierte exklusive Rolle von NK1.1⁺ T-Zellen als Lieferanten des frühen IL-4 in der Maus wurde durch Experimente mit genetisch defizienten Tieren in Frage gestellt [Coffman,R.L. et al., J. Exp. Med. 185 (1997) 373-375].

Verschiedene Autoren haben vermutet, dass Basophile in die Entstehung einer Th2-Antwort involviert sind [Paul, W.E. et al., Cell 76 (1994) 241-251; Romagnani,S., Immunol. Today 13 (1992) 379-381; Dahinden,C.A., Int. Arch. Allergy Immunol. 113 (1997) 134-137]. Dafür spricht, dass menschliche Basophile innerhalb weniger Stunden beträchtliche Mengen an IL-4 nach Antigen-spezifischer und unspezifischer Stimulation freisetzen [Brunner,T. et al., J. Exp. Med. 177 (1993) 605-611; Kasaian,M.T. et al., Int. Immunol. 8 (1996) 1287-1297]. Weiterhin können Basophile als bewegliche Zellen des peripheren Blutes schnell an Orten der Auseinandersetzung mit Pathogenen akkumulieren. Wanderung und Akkumulation werden durch eine Reihe von Chemokinen gesteuert. Besonders hervorzuheben ist Eotaxin, das Basophile, Eosinophile und Th2-Zellen durch Bindung an deren Chemokinrezeptor CCR3 anlockt. Interessanterweise führt IL-4 zur Freisetzung von Eotaxin aus menschlichen Hautfibroblasten und Endothelzellen, was einen Amplifikationsmechanismus für die Rekrutierung von Basophilen darstellen dürfte. Neben seiner chemotaktischen Wirkung verstärkt Eotaxin deutlich die Antigen-induzierte IL-4-Freisetzung aus Basophilen. Verglichen mit Basophilen ist die IL-4-Produktion durch Mastzellen und Eosinophile niedrig, was deren Rolle bei der Th2-Induktion relativiert. Zusammenfassend kommen somit vor allem Basophile als Produzenten des frühen IL-4 in Betracht.

Die Frage nach den Triggerfaktoren für das frühe IL-4 ist bis heute nicht sicher beantwortet. Für das frühe IL-12 kennt man dagegen bereits auslösende Faktoren. So können z.B. bakterielle Produkte, wie Lipopolysaccharid und CpG-Oligodesoxynucleotide, Makrophagen oder dendritische Zellen zur raschen Freisetzung von IL-12 veranlassen und eine Th1-Induktion *in vivo* bewirken [Paul,W.E. et al., Cell 76 (1994) 241-251; Bohle,B. et al., Eur. J. Immunol. 29 (1999) 2344-2353]. Die Identifikation von Faktoren für die Th2-Induktion steht noch aus. Allerdings wurden bereits potentielle Triggerfaktoren beschrieben, die *in vitro* eine rasche IL-4-Freisetzung aus Basophilen induzieren: die B-Zell-Superantigene Protein Fv [Patella,V. et al., J. Immunol. 161 (1998) 5647-5655] und HIV-Glykoprotein 120 [Patella,V. et al., J. Immunol. 164 (2000) 589-595], die durch Bindung an das VH3-Segment von IgE wirksam werden, sowie Lektine [Haas,H. et al., Eur. J. Immunol. 29 (1999) 918-927], die an die Kohlenhydratseitenketten von IgE oder des IgE-Rezeptors binden.

Zur Therapie bestimmter Erkrankungen wäre es wünschenswert, die Immunantwort in Richtung einer T-Helferzellantwort vom Typ2 (Th2-Antwort) zu lenken. Hierfür gibt es bisher noch keine befriedigenden Möglichkeiten, und die Gabe von IL-4 als solchem ist für den routinemäßigen Einsatz nicht praktikabel, da sie zu aufwendig und zu teuer ist.

Aufgabe der vorliegenden Erfindung ist es daher, hochpotente Th2-Induktoren sowie Arzneimittel zur Immunmodulation zur Verfügung zu stellen.

Die Aufgabe wird erfindungsgemäß durch neue Proteine gelöst, die aus parasitären Würmern isoliert werden.

Parasitäre Würmer lassen sich im wesentlichen drei großen taxonomischen Gruppen von Helminthen zuordnen (Zestoden, Trematoden und Nematoden) und zeichnen sich aus durch eine parasitäre Lebensweise innerhalb von Wirbeltieren und/oder Wirbellosen. Dort befallen sie jeweils bestimmte Organe/Gewebe: das Darmlumen (Darmnematoden), Epithelien (Lungenwürmer), Blutgefäße (Schistosomen), Lymphgefäße und Haut (Filarien) sowie verschiedene Körpergewebe (Larvenstadien von Bandwürmern) und sind dabei mit unterschiedlichen immunologischen Bedingungen konfrontiert.

Weltweit sind ca. 3,5 Milliarden Menschen mit parasitären Würmern infiziert. Auch wenn die direkte Mortalität niedrig ist, können die Betroffenen dadurch beträchtliche Gedeih-, Entwicklungs- und Organschäden sowie oft chronisches Siechtum erfahren.

Wichtigste Vertreter parasitärer Würmer beim Menschen: Spulwurm (Ascaris lumbricoides; ca. 1 Milliarde Menschen infiziert), Hakenwürmer (Ankylostoma duodenale und Necator americanus; knapp 1 Milliarde), Peitschenwurm (Trichuris trichiura; ca. 800 Millionen), Schistosomen (ca. 200 Millionen), Filarien (Brugia, Onchocerca und Wuchereria, ca. 100 Millionen) und Bandwurmarten (Taenia; ca. 50 Millionen).

Bei Tieren ist die Durchseuchung mit parasitären Würmern, von denen es sehr viele verschiedene Arten gibt, noch weitaus höher als beim Menschen. Der wirtschaftliche Schaden bei Nutztieren kann erheblich sein.

Die Erfindung beruht auf der Erkenntnis, dass vor allem parasitäre Würmer zuverlässig eine Th2-Antwort induzieren. Dabei kann die Eigenschaft der Th2-Induktion auf ein bestimmtes Entwicklungsstadium beschränkt sein. So induziert bei Infektion von Mäusen mit dem Trematoden *Schistosoma mansoni* das Ei-Stadium eine Th2-artige Immunreaktion, während das Schistosomula-(Larven-)Stadium eine Th1-artige Immunreaktion auslöst [Pearce,E.J. et al., J. Exp. Med. 173 (1991) 159-166]. Die IL-4-induzierende Potenz der Schistosomeneier ist so ausgeprägt, dass auch naive Mäuse nach transienter Th0-Antwort innerhalb von 7-10 Tagen mit einer Th2-Antwort und IgE-Synthese auf die Gabe von Schistosomeneiern reagieren [Vella,A.T. et al., J. Immunol. 148 (1992) 2283-2290]. Bereits nach intranasaler Applikation von *S. mansoni*-Ei-Extrakt kommt es im Mausmodell zu einer systemischen IgE-Produktion [Okano,M. et al., J. Immunol. 163 (1999) 6712-6717]. Bislang ist es jedoch nicht gelungen, den oder die Wirkstoffe zu identifizieren oder zu isolieren, die für die ausgeprägte IL-4-induzierende Kapazität von *S. mansoni*-Eiern verantwortlich sind.

### Detaillierte Beschreibung der Erfindung:

Im Rahmen der vorliegenden Erfindung wird nunmehr erstmals ein Verfahren zur Verfügung gestellt, mit dem die Isolierung der IL-4 induzierenden Substanzen aus *S. mansoni*-Eiern sowie aus Eiern anderer parasitärer Würmer möglich ist.

Gegenstand der Erfindung ist daher ein Verfahren zur Isolierung eines IL-4 induzierenden Proteins aus parasitären Würmern, bei dem man Ei-Antigen (EA) oder Extrakt aus Th2- bzw. IgEinduzierenden Stadien parasitärer Würmer (EX) gegen Wasser dialysiert und lyophilisiert, man das lyophilisierte EA od EX mittels Kationenaustausch-Chromatographie auftrennt, man die Fraktionen, die bei Inkubation humaner basophiler Granulozyten eine IL-4-Induktion bewirken, poolt, einengt, man eine Affinitätschromatographie mit NHS-aktivierter Sepharose durchführt, an die *Aleuria aurantia*-Agglutinin gekoppelt ist, man die Fraktionen, die bei Inkubation humaner basophiler Granulozyten eine IL-4-Induktion bewirken, poolt und einengt. Vorzugsweise lyophilisiert man das erhaltene Protein anschließend.

Gemäß einer bevorzugten Ausführungsform der Erfindung verwendet man EA von *Schistosoma mansoni, Schistosoma japonicum, Schistosoma haematobium, Fasciola hepatica, Dicrocoelium lanceolatum, Echinococcus multilocularis, Ascaris lumbricoides, Ascaris suum, Ankylostoma duodenale, Necator americanus, Trichuris trichiura, Brugia malayi, Onchocerca volvulus, Wuchereria bancrofti, Taenia solium, Taenia bovis* und verwandten Arten.

Als Alternative zu *A.* aurantia-Agglutinin werden monoklonale Antikörper gegen die erfindungsgemäßen Proteine und Polypeptide (Protein- und Kohlenhydratkomponenten) hergestellt, die zur Aufreinigung verwandter Moleküle anderer Wurmspezies herangezogen werden.

Diese monoklonalen Antikörper werden auch für immunhistologische Untersuchungen eingesetzt, so z.B. zur Lokalisation der erfindungsgemäßen Proteine und Polypeptide und daraus abgeleiteten Molekülen im Parasiten oder um die Interaktion dieser Proteine, Polypeptide oder abgeleiteten Moleküle mit Wirtszellen zu charakterisieren. Weiterhin können diese Antikörper - u.U. in humanisierter Form - zur biologischen Neutralisation des Effekts der genannten Proteine, Polypeptide und daraus abgeleiteter Moleküle bzw. damit verwandter Molekülen (wie Allergenen) *in vitro* und *in vivo* (letzteres bei Zuständen einer unerwünschten Th2-/IgE-Immunantwort) Verwendung finden.

Gemäß einer besonderen Ausführungsform der Erfindung nimmt man das lyophilisierte EA oder EX zur Durchführung der Kationenaustausch-Chromatographie in 20 mM Kaliumphosphatpuffer bei einem pH-Wert von 5,0 auf. Gemäß einer weiteren besonderen Ausführungsform der Erfindung puffert man die aus der Kationenaustausch-Chromatographie erhaltenen aktiven Fraktionen nach dem Poolen und Einengen auf 50 mM Natriumphosphatpuffer, pH-Wert 7,0, um, bevor man die Affinitätschromatographie durchführt.

Bei Verwendung von *Schistosoma mansoni*-Ei-Antigen (SmEA) erhält man auf diese Weise ein als IPSE bezeichnetes Protein, das die in SEQ ID NO: 2 gezeigte Aminosäuresequenz aufweist. Das Protein ist vorzugsweise glykosyliert, es weist jedoch auch in nichtglykosylierter Form ausgeprägte IL-4-induzierende Eigenschaften auf.

Als "SEQ ID NO" wird vorliegend die Numerierung nach der im WIPO-Standard ST.25 verwendeten numerischen Kennzahl <400> verstanden.

Gegenstand der Erfindung sind ferner Proteine oder Polypeptide, die Homologe, Derivate oder Fragmente der erfindungsgemäßen Proteine und Polypeptide und insbesondere von IPSE sind, die ebenso die Aktivität aufweisen, bei humanen basophilen Granulozyten die Produktion von IL-4 und/oder IL-13 zu stimulieren. Unter Homologen sind vorliegend solche Proteine oder Polypeptide zu verstehen, die gegenüber der in SEQ ID NO: 2 dargestellten Sequenz ein oder mehrere Aminosäureaustausche aufweisen, unter Derivaten sind solche Moleküle zu verstehen, bei denen einzelne Aminosäuren beispielsweise durch nicht natürliche (artifizielle) Aminosäuren oder Aminosäuren in der D-Form ersetzt sind.

Von Okano et al. wurde vorgeschlagen, daß die Kohlenhydrat-Komponenten von *S. mansoni*-Ei-Antigenen (SmEA) die Th1-Antworten herunterregulieren und auch für die SmEA-stimulierte Th2-Induktion erforderlich sind (vgl. Velupillai, P. et al., Proc. Natl. Acad. Sci. USA 91 (1994) 18-22, Okano et al., J. Immunol. 163 (1999) 6712-6717). Die Autoren führten den Nachweis an einem Mausmodell bei intranasaler Sensitivierung mit deglykosyliertem (Parjodat-behandaltem) SmEA, das - im Gegensatz zum nativen Ei-Antigen - nicht in der Lage war, IL-4, IL-5, IL-10 und die SmEAspezifische IgE-Produktion zu induzieren. Ferner wurde festgestellt, daß Lacto-N-Fucopentaose III, eine prädominante Glykan-Komponente von SmEA, die Lewis^{x} (Le^{x}) enthält, als Th2-induzierendes Adjuvans in Mäusen wirkt, wenn es kovalent an humanes Serumalbumin gebunden ist (M. Okano et al., J. Immunol. 167 (2001) 442-450).

Im Rahmen der vorliegenden Erfindung wurde festgestellt, daß die erfindungsgemäßen Proteine bzw. Polypeptide, insbesondere IPSE, auch in Abwesenheit von Kohlehydraten, d.h. in unglykosylierter Form, funktionell wirksam sind. Insbesondere weist unglykosyliertes, rekombinantes IPSE einen starken IL-4-induzierenden Effekt auf. Die im Rahmen der vorliegenden Erfindung erhaltenen Ergebnisse schließen jedoch nicht die Möglichkeit aus, daß Kohlenhydrate zu einer Th2-Ausrichtung beitragen, wobei es andere Zelltypen als Basophile einschließen könnte, die von Fucopentaose III-gekoppeltem humanem Serumalbumin nicht aktiviert werden.

Erfindungsgemäß eingeschlossen sind daher ferner die oben genannten Proteine oder Polypeptide in unglykosylierter Form, da festgestellt wurde, dass diese einen ebenso wirksamen IL-4-induzierenden Effekt aufweisen wie deren glykosylierte Analoga.

Die Erfindung betrifft ferner ein Nukleinsäuremolekül, das eine für ein oben genanntes Protein oder Polypeptid kodierende Nukleinsäuresequenz aufweist wobei es sich nicht um ein Molekül mit folgender Sequenz handelt: Database EM_EST Eintrag AI820476 (Oliveira et al.) offenbart - ohne Hinweis auf eine evtl. Aktivität kodierter Polypeptide - diese Expressed Sequence Tag (EST)-Sequenz aus *Schistosoma mansoni*, welche im Überlappungsbereich eine hohe Identität mit SEQ ID NO: 1 aufweist. Gemäß einer besonderen Ausführungsform der Erfindung weist das Nukleinsäuremolekül die in SEQ ID NO: 1 gezeigte Nukleinsäuresequenz auf.

### Vorteile der Erfindung:

Die erfindungsgemäßen Proteine und Polypeptide führten bereits in sehr niedriger Konzentration zur Freisetzung der zellulären Botenstoffe Interleukin-4 (IL-4) und Interleukin-13 (IL-13) aus basophilen Granulozyten (weißen Blutkörperchen) nicht-sensibilisierter gesunder Blutspender. IL-4 und IL-13 sind Schlüsselfaktoren für die Entstehung der Immunglobulin-E-vermittelten (IgE-vermittelten) Allergie. Die erfindungsgemäßen Proteine spielen eine zentrale Rolle bei der für parasitäre Würmer typischen Weichenstellung zur T-Helferzell-Antwort vom Typ2 (Th2-Antwort) und zur Synthese von IgE. Da Th2-induzierende Faktoren die Entstehung einer Th1-Antwort (zelluläre Immunantwort) hemmen, können die hochpotenten Moleküle der vorliegenden Erfindung immunpharmakologisch eingesetzt werden, z.B. bei Erkrankungen die mit einer pathologischen Th1-Antwort einhergehen, wie der Multiplen Sklerose.

Gegenstand der Erfindung ist daher die Verwendung des erfindungsgemäßen Proteins oder Polypeptids zur Herstellung einer pharmazeutischen Zusammensetzung zur Immunmodulation, vorzugsweise zur Herstellung einer pharmazeutischen Zusammensetzung zur Stimulation der IL-4- und/oder IL-13-Produktion bzw. zur Herstellung einer pharmazeutischen Zusammensetzung zur Stimulation der IgE-Produktion.

Die Induktion oder Verstärkung einer Th2-betonten Immunantwort ist auch im Hinblick auf Entwicklung bestimmter Impfstoffe wünschenswert. So ist eine Th2-Antwort bei einigen parasitären Wurminfektionen protektiv (z.B. bei Nematoden-Infektionen oder der Schistosomiasis), während hier eine Th1-betonte Immunantwort den gegenteiligen Effekt hat. Dies ist bei der Herstellung anthelminthischer und anderer Vakzinen zu berücksichtigen.

Untersuchungen hinsichtlich des Wirkungsmechanismus zeigten, dass die Basophilenaktivierung (Degranulation, Histamin-, IL-4- und IL-13-Freisetzung) durch IPSE die Anwesenheit von IgE an der Oberfläche der Basophilen erfordert. Dies ergaben sogenannte IgE-Stripping- und IgE-Resensibilisierungs-Experimente, bei denen Rezeptor-gebundenes IgE durch kurzfristige pH-Erniedrigung von den Basophilen abgelöst wird, und die Zellen anschließend wieder erneut im IgE beladen werden. Die IgE-Abhängigkeit der IL-4-Induktion lässt vermuten, dass IPSE durch Bindung und Quervernetzung von Rezeptor-gebundenem IgE wirksam wird.

Um zu prüfen, ob es sich bei IPSE um einen Immunglobulin-bindenden Faktor handelt, wurden Western- und Dotblotting-Techniken eingesetzt. Dabei wurden IPSE bzw. IgE mittels Blotting an Nitrozellulose-Membran fixiert und der jeweilige Partner in der löslichen Phase zugesetzt. Unter beiden Bedingungen kam es zur Bindung zwischen IgE und IPSE. Erste Untersuchungen mit dem Biacore-Verfahren (Surface-Plasmon-Resonanz; Amersham Pharmacia Biotech, Freiburg, Deutschland) bestätigten diesen Befund, wobei sich eine hohe Bindungsaffinität zwischen IPSE und IgE nachweisen ließ. Um zu ermitteln, ob die Immunglobulin-bindende Aktivität von IPSE auf den IgE-Isotyp beschränkt ist oder auch andere Immunglobulinklassen betrifft, wurden Westernblot-Untersuchungen mit IgG aus Seren gesunder nordeuropäischer Blutspender durchgeführt. Dabei stellte sich heraus, dass IPSE auch die menschlichen IgG-Subklassen 1-4 bindet. Interessanterweise ergaben kompetitive Inhibitionsversuche, dass IPSE an IgE mit etwa 100fach höherer Affinität bindet als an IgG.

Darüber hinaus bindet IPSE an alle Peptidfragmente von IgG nach Papainverdau, d.h. sowohl an die Fab- als auch vor allem an die Fc-Fragmente.

Aufgrund seiner Eigenschaften als Immunglobulin-bindender Faktor kann man die erfindungsgemäßen Polypeptide, insbesondere IPSE, daher zum Nachweis und zur Aufreinigung von Immunglobulinen, insbesondere von IgE und IgG, sowie von Fragmenten derselben (wie Fc- und/oder Fab-Fragmenten), verwenden, beispielsweise bei der Durchführung eines ELISA (enzyme linked immunosorbent assay) oder im Rahmen einer Affinitätschromatographie.

Aufgrund der immunologischen Eigenschaften der Proteine und Polypeptide der Erfindung eignen sich diese in besonderem Maße zur Herstellung einer pharmazeutischen Zusammensetzung zur Induktion oder Verstärkung einer Th2-Immunantwort, insbesondere zur Herstellung einer pharmazeutischen Zusammensetzung zur Behandlung von Erkrankungen, die mit einer Th1-Immunantwort einhergehen. Vorzugsweise handelt es sich bei diesen Erkrankungen um Erkrankungen aus der Gruppe bestehend aus Multipler Sklerose, autoimmuner Uveitis, Diabetes mellitus, rheumatoider Arthritis, Behcets Syndrom, Helicobacter pylori-Infektion, entzündlichen Darmerkrankungen (insbesondere Morbus Crohn), akuter Organtransplantat-Abstroßungsreaktion und spontanen rekurrenten Aborten.

Insbesondere ist Gegenstand der vorliegenden Erfindung die Verwendung der erfindungsgemäßen Polypeptide, vor allem aber von IPSE, zur Herstellung einer pharmazeutischen Zusammensetzung zur Immunmodulation, beispielsweise bei allergischen Reaktionen. Aufgrund seiner Immunglobulin-bindenden Eigenschaften sind die erfindungsgemäßen Peptide im übrigen für die Therapie von Erkrankungen geeignet, die durch einen erhöhten Serum-IgE Spiegel und/oder eine vermehrte Produktion von Interferon-gamma charakterisiert sind, wie z.B. die allergische Rhinokonjunktivitis und das allergische Asthma bronchiale.

Eingeschlossen sind ferner pharmazeutische Zusammensetzungen, die ein erfindungsgemäßes Protein oder Polypeptid zusammen mit pharmazeutisch annehmbaren Hilfs- und Trägerstoffen enthalten.

Die Proteine und Polypeptide der vorliegenden Erfindung dienen ferner zur Induktion oder Verstärkung der IL-4-, IL-13- und/oder IgE-Produktion *in vitro*, wobei man speziell humane basophile Granulozyten, B-Zellen, T-Zellen oder natürliche Killer (NK)-Zellen, aber auch andere Zellen (des Immunsystems oder anderer Gewebe, wie z.B. Endothelzellen oder Fibroblasten) sowie daraus abgeleitete Zelllinien mit einem oben genannten Protein oder Polypeptid inkubiert. Damit sind die Proteine und Polypeptide zur Herstellung von IL-4 und/oder IL-13 in vitro geeignet. Bei diesem Verfahren inkubiert man die oben genannten Zellen mit dem Protein oder Polypeptid und isoliert IL-4 und/oder IL-13 anschließend aus dem Kulturüberstand.

Eine weitere Verwendung der erfindungsgemäßen Proteine und Polypeptide besteht in der Hemmung der Th1-Zytokinproduktion/Th1-Antwort durch mononukleäre Zellen aus peripherem Blut (von Mensch und Tier) *in vitro*.

Da die oben genannten Nukleinsäuremoleküle für die erfindungsgemäßen Proteine und Peptide kodieren, eignen sie sich als Matritze zur *in vitro*-Herstellung der Proteine bzw. Polypeptide, d.h. zur *in vitro*-Expression. Hierzu wird die DNA beispielsweise in einen Expressionsvektor eingebaut und die entsprechenden Proteine rekombinant in geeigneten Wirtszellen, wie z.B. *Escherichia coli*, überexprimiert. Falls die Glykosylierung für die funktionelle Aktivität der Proteine oder Polypeptide kritisch ist, erfolgt die Expression im Baculovirus- oder einem anderen eukaryontischen System.

Gegenstand der Erfindung ist daher ferner ein Expressionsvektor, der ein erfindungsgemäßes Nukleisäuremolekül eingebaut enthält.

Die Nukleinsäuremoleküle eignen sich ferner zur Herstellung einer pharmazeutischen Zusammensetzung zur *in vivo*-Expression (z.B. DNA-Vakzinierung). Die Herstellung der oben genannten Proteine und Polypeptide kann somit auch *in vivo* erfolgen und zwar vor allem in Hinblick auf eine Umstimmung der Immunantwort. Hierzu wird die entsprechende DNA in einen Gentransfervektor (wie z.B. einen adenoviralen Vektor) eingebaut. So führen z.B. adenovirale Expressionssysteme zu einer transienten, sehr hohen Proteinproduktion; auch organspezifische Expression ist möglich. Durch intramuskuläre Injektion des Vektors kann - zunächst bei Versuchstieren - eine lokale Protein- bzw. Polypeptid-Produktion ausgelöst werden. Geeignete Expressionssysteme, insbesondere für die Anwendung am Menschen, sind dem Fachmann wohlbekannt und werden hinsichtlich ihrer Anwendungssicherheit laufend verbessert.

Gegenstand der Erfindung ist daher auch ein Gentransfervektor, in den ein erfindungsgemäßes Nukleinsäuremolekül eingebaut ist, sowie eine pharmazeutische Zusammensetzung, die einen solchen Gentransfervektor zusammen mit pharmazeutisch annehmbaren Hilfs- und/oder Trägerstoffen enthält.

Im folgenden wird die Erfindung anhand von Ausführungsbeispielen näher erläutert.

### Beispiel 1

### Methode zur Gewinnung von Schistosoma mansoni-Ei-Antigen (SmEA)

Das Ausgangsmaterial für die Aufreinigung von IPSE sind Eier von *Schistosoma mansoni,* die nur in limitierter Menge zur Verfügung stehen, da ihre Gewinnung aufwendig und teuer ist. Aus den Schistosomen-Eiern wurde durch Extraktion in Phosphat-gepufferter Kochsalzlösung ein Gesamtextrakt gewonnen (Boros DL et al., J. Exp. Med. 132 (1970) 488-507; Carter CE et al., J. Parasitol. 64 (1978) 285-290), das sog. *Schistosoma mansoni*-Ei-Antigen (SmEA). Dieses wurde nach Dialyse gegen destilliertes Wasser in lyophilisierter Form bis zur weiteren Verarbeitung aufbewahrt. Da IPSE nur in sehr geringer Konzentration in SmEA enthalten ist, ist es nach Darstellung des Gesamtextrakts durch SDS-PAGE und Silberfärbung unter den zahlreichen Bestandteilen nicht zu erkennen.

### Beispiel 2

### Methode zur Aufreinigung von IPSE aus SmEA

### A. Kationenaustauschchromatographie:

Im Gegensatz zu anderen Verfahren zur Proteinreinigung wie Ausschlusschromatographie (Superdex 200 PC, SMART-System, Pharmacia), hydrophobe Interaktionschromatographie (HIC), Reversed Phase-HPLC und präparative Isoelektrofokussierung (Rotofor, Bio-Rad Laboratories, München, Deutschland), die in unseren Händen zu erheblichem bis totalem Verlust und/oder unbefriedigender Anreicherung des IL-4-induzierenden aktiven Prinzips führten, gelang uns mit der Technik der Kationenaustauschchromatographie eine deutliche Anreicherung von IPSE.

Dazu wurde lyophilisiertes SmEA (2 mg) in 0,5 ml 20 mM Kaliumphosphatpuffer, pH 5,0, aufgenommen und mittels Kationenaustauschchromatographie (SP-Sepharose; vorgepackte 1-ml HiTrap^{®}-Säulen, Amersham Pharmacia, Freiburg, Deutschland) aufgetrennt. Nach dem Auftragen der Probe und Waschen mit Startpuffer (20 mM K-phosphatpuffer, pH 5,0) wurde gebundenes Material mit einem linearen Salzgradienten bis 1M KCl in Startpuffer eluiert. Die Auftrennung erfolgte auf einer *Äktaprime*-Anlage (Amersham Pharmacia, Freiburg, Deutschland) mit einer Laufmittelgeschwindigkeit von 1 ml/min. Es wurden Fraktionen von 0,5 ml gesammelt. Der pH von 5,0 ist kritisch, da bei höheren pH-Werten (pH 6,0) ein Großteil von IPSE zusammen mit dem Protein-reichen Effluent die Säule verlässt und somit die Anreicherung des aktiven Prinzips unterbleibt. Anschließend wurden die funktionell aktiven Fraktionen (Nachweis über Ermittlung der IL-4-Induktion im Basophilentest, s.u.) gepoolt und mittels Centricon Plus20 (Millipore, Eschborn, Deutschland) eingeengt und auf 50 mM Natriumphosphatpuffer, pH 7,0 umgepuffert. Die Verwendung von Centricon Plus20 zur Einengung war kritisch für die Ausbeute an IPSE, da es bei Benutzung anderer Konzentrationseinheiten wie z.B. Centricon und Centriprep zu einem Verlust des aktiven Prinzips von über 90% kam. Um die Verluste bei Einengung mit Centricon Plus20 noch weiter zu vermindern, wurden die Einheiten zusätzlich mit 5 % Tween 20 über Nacht vorinkubiert. Vor Benutzung wurden sie dann gut mit reichlich Aqua bidest. gespült, um eine Verschleppung von Tween in die funktionellen Assays und damit eine Schädigung der Basophilen mit der Folge falsch negativer Ergebnisse zu vermeiden. Dieses Vorgehen gestattete es, den Verlust an IL-4-induzierender Aktivität im Rahmen der Einengungsprozedur unter 50% zu halten.

Nach Einengung der Proben (um mindestens Faktor 5) gelang es, in den aktiven Fraktionen neben irrelevanten Komponenten zwei benachbarte Banden im Molekulargewichtsbereich um 40 kD im SDS-PAGE-Gel nach Silberfärbung zu erkennen. Für den Nachweis dieser beiden Banden war jedoch eine längere Entwicklungszeit der Silberfärbung (mindestens 30 min) erforderlich. In den nicht-aktiven Fraktionen war die Doppelbande nicht nachweisbar.

Der Verdacht, dass es sich bei einer der beiden Banden (oder beiden) um das gesuchte aktive Prinzip handelte, wurde durch das Ergebnis des folgenden Experiments verstärkt: Auftrennung von SmEA über SDS-PAGE und fraktionierte Elektroelution des Gels ließ die IL-4-induzierende Aktivität dem Molekulargewichtsbereich um 40 kD zuordnen, d.h. dem Bereich in dem sich die beiden Banden befanden.

### B. Affinitätschromatographie über Aleuria aurantia-Agglutinin-Sepharose:

Zur weiteren Aufreinigung wurde die Tatsache genutzt, dass Schistosomen-Eier zahlreiche, z.T. ungewöhnlich glykosylierte Glykoproteine und Glykolipide enthalten. Dementsprechend zeigte eine Affino-Blot-Analyse, dass die Komponenten des Schistosomen-Ei-Gesamtextraktes nach Trennung über SDS-PAGE und Transfer auf Nitrozellulose-Membran differentiell von einer Reihe markierter Lektine erkannt wurden, u.a. von Aleuria aurantia-Agglutinin, Concanavalin A, Datura *stramonium*-Agglutinin, *Galanthus nivalis-*Agglutinin, *Maackia amurensis*-Agglutinin, Peanut-Agglutinin, *Ricinus communis*-Agglutinin, *Sambucus nigra*-Agglutinin, Weizenkeim-Agglutinin. Mit den aktiven Fraktionen nach Kationen-Austauschchromatographie reagierten dagegen nur noch drei Lektine. Von diesen interagierte nur *Aleuria aurantia*-Agglutinin, das an α1-6-glykosidisch verknüpfte Fucose bindet, selektiv mit den beiden benachbarten Banden um 40 kD. Demgegenüber reagierten die beiden anderen Lektine (Peanut-Agglutinin und *Ricinus communis-*Agglutinin) ausschließlich bzw. zusätzlich mit anderen, auch in nicht-aktiven Fraktionen nachweisbaren Banden.

Es wurde daher die Arbeitshypothese aufgestellt, dass es sich bei der Doppelbande um IPSE handelt, und als nächster Schritt wurde eine Affinitätschromatographie mit *Aleuria aurantia-*Agglutinin (Vector Laboratories, Alexis-Deutschland GmbH, Grünberg, Deutschland) durchgeführt. Hierzu wurde dieses Lektin an NHS-aktivierte Sepharose (HiTrap Säule; Amersham Pharmacia) gekoppelt. Die Kopplung erfolgte gemäß den Empfehlungen des Herstellers. Zur Affinitätschromatographie wurden die funktionell aktiven Fraktionen aus der Kationenaustauschchromatographie auf die Säule gegeben, mit Startpuffer (50 mM Natriumphosphatpuffer, pH 7,0) gewaschen und mit Elutionspuffer (Startpuffer + 0,1 M Fucose (Sigma, Taufkirchen, Deutschland)) eluiert. Anschließend wurden die Fraktionen wiederum mittels Centricon Plus20 (Millipore GmbH, Eschborn, Deutschland), wie oben beschrieben, eingeengt und das gewonnene Material mit Hilfe von SDS-PAGE und anschließender Silberfärbung oder anschließendem Affinoblotting mit markiertem *A. aurantia*-Agglutinin erneut charakterisiert. Die IL-4-induzierende Aktivität befand sich ausschließlich in den Fraktionen mit der *A. aurantia*-Agglutininbindenden Doppelbande. Die positiven Fraktionen wurden gepoolt und portioniert oder gegen Aqua bidest. dialysiert und lyophilisiert. Es folgte Lagerung bei -80°C.

### Beispiel 3

### Ermittlung der kompletten Nukleotid- und Aminosäuresequenz von IPSE

### A. N-terminale Ansequenzierung von IPSE:

Zur Gewinnung ausreichender Mengen an IPSE für die N-terminale Sequenzierung wurden die aktiven Fraktionen aus 10 Läufen der Kationenaustauschchromatographie gepoolt und über die *Aleuria aurantia*-Agglutinin-Affinitätschromatographie weiter aufgereinigt. Das auf diese Weise gewonnene Material (IPSE) kam in der SDS-PAGE (Silberfärbung bzw. Lektinblot) bei 40 kD als Doppelbande ohne weitere Kontaminanten zur Darstellung. Die N-terminale Ansequenzierung beider Banden (ohne weitere Auftrennung) gestattete die Identifikation von 18 der 20 N-terminalen Aminosäuren. Die Suche in Proteindatenbanken ergab keine derzeit bekannten homologen Proteine. Die Suche in einer EST-Datenbank erbrachte an erster Stelle ein *Expressed* Sequence *Tag* (EST) aus einer *Schistosoma mansoni-*Ei-cDNA-Bank. Aus einem der drei Leseraster dieses EST ließ sich eine Aminosäuresequenz ableiten, die in 11 benachbarten Aminosäuren mit der N-terminalen IPSE-Sequenz identisch war.

### B. Isolierung der cDNA für IPSE aus S. mansoni-Ei-cDNA-Banken

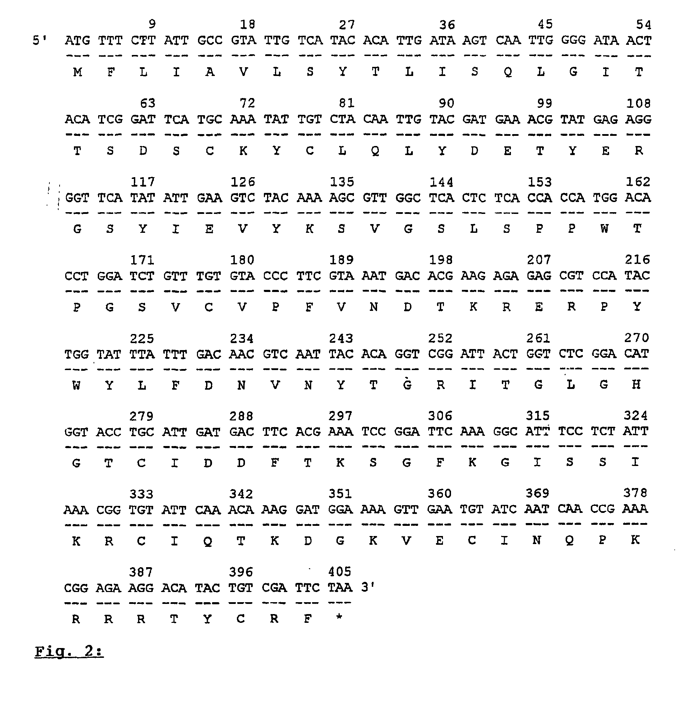

Die im vorherigen Abschnitt beschriebene Sequenzinformation gestattete die Herstellung einer spezifischen (nicht-degenerierten) DNA-Sonde zur Suche nach der Nukleotidsequenz von IPSE in *Schistosoma mansoni*-Ei-cDNA-Banken. Sowohl in einer von uns als auch in einer von G. Oliveira hergestellten *S. mansoni*-Ei-cDNA-Bank (beide im λ-Zap-Vektor) fanden sich Klone, die mit dieser Sonde reagierten. Die Sequenzierung dieser Klone ergab, dass ihre gesamte N-terminale Sequenz mit der N-terminalen Sequenz von IPSE identisch ist, wobei es sich bei den beiden zunächst unbekannten Aminosäuren um Cysteine handelte (die Aminosäure Cystein ist via N-terminale Sequenzierung schwer nachweisbar). Ein Vergleich der im Rahmen der vorliegenden Erfindung gefundenen Sequenz mit dem oben angegebenen EST ergab erhebliche Unterschiede, die möglicherweise auf Fehler bei der Sequenzierung und Sprünge im Leseraster des EST zurückzuführen sind. In Fig. 2 ist die komplette Sequenz von IPSE auf DNA-Ebene sowie auf Protein-Ebene dargestellt (mit einer Leader-Sequenz von 20 Aminosäuren).

### Beispiel 4

### Nachweissystem für IPSE

### A. Reinigung und Stimulation menschlicher basophiler Granulozyten:

Die Isolierung und Stimulation menschlicher basophiler Granulozyten erfolgte nach der Methode von K. Haisch et al. (J. Immunol. Methods 226 (1999) 129-137). Dazu wurden mit Hilfe eines Ficoll/Percoll-Gradienten aus Venenblut gesunder Blutspender mononukleäre Zellen gewonnen, aus denen über Gegenstrom-Elutriation Basophilen-reiche Fraktionen isoliert wurden. Mittels immunmagnetischer Beads (MACS-System) wurden daraus die Basophilen hochrein isoliert (Routinemäßig über 98 % Reinheit). Die gereinigten Basophilen wurden mit SmEA bzw. seinen Fraktionen für 4 h (IL-4) bzw. 18 h (IL-13) inkubiert und die Überstände für die Zytokinbestimmung abgenommen und bis zur Untersuchung bei -70°C aufbewahrt.

### B. IL-4 und IL-13-ELISA:

Die Konzentrationen der Zytokine IL-4 und IL-13 im Kulturüberstand wurden mittels eines Sandwich-ELISAs bestimmt (u.a. kommerziell erhältlich).

### Beispiel 5

### Gewinnung polyklonaler und monoklonaler Antikörper gegen IPSE

Zur Gewinnung polyklonaler Antikörper wird IPSE in Anwesenheit von TiterMax-Adjuvans Kaninchen intramuskulär in beide Schenkel injiziert. Die Bildung von IPSE-spezifischen Antikörpern wird im Westernblot ermittelt. Boosterinjektionen erfolgen in 4-wöchigen Abständen.

Die Herstellung monoklonaler Antikörper erfolgt nach der von Köhler und Milstein entwickelten Methode (Nature 256 (1975) 495-497) in der Modifikation nach Current Protocols of Immunology (Editors: J.E. Coligan et al., Herausgeber: John Wiley & Sons, Inc. (1997); ISBN: 0-471-522767). Dazu wird IPSE in Anwesenheit von Adjuvans zwei Mäusen intraperitoneal injiziert. Eine Boosterinjektion (ohne Adjuvans) erfolgt nach 10-14 Tagen. 3 Tage später werden die Milzzellen der Mäuse mit der Myelomzelllinie SP2/0-Ag14 fusioniert. Am nächsten Tag wird Hypoxanthin-Aminopterin-Thymidin (HAT)-haltiges Medium zur Selektion der Hybridomzellen zugesetzt. Nach 10-14 Tagen erfolgt das Screening der primären Hybridomüberstände auf Antikörper-Reaktivität gegenüber IPSE im Westernblotverfahren. Kandidaten-Hybridom-Linien werden propagiert und durch *Limiting Dilution* re/kloniert.

### Beispiel 6

### Wirksamkeit von nicht-glykosyliertem IPSE

Lacto-N-Fucopentaose III (LNFPIII) ist eine Hauptglykankomponente von Glykoproteinen in *S. mansoni-*Ei-Antigen-Extrakten. Vor dem Hintergrund der Eigenschaft der erfindungsgemäßen Polypeptide, insbesondere IPSE, humane Basophile zu aktivieren, wurde daher untersucht, ob an humanes Serumalbumin gebundene Lacto-N-Fucopentaose III (LNFPIII-HSA) (Biocarb Chemicals, Lund, Schweden) ebenfalls in der Lage war, Basophile zu aktivieren. Das experimentelle Vorgehen entsprach der in Beispiel 4 A genannten Methode.

Es wurde festgestellt, daß im Gegensatz zu dem als Positivkontrolle verwendeten *S. mansoni*-Ei-Antigen LNFPIII-HSA über einen weiten Konzentrationsbereich (0,03 - 30 µg/ml) die Degranulation von Basophilen, die Freisetzung von Mediatoren oder die Expression von IL-4 oder IL-13 nicht induzierte.

### Beispiel 7

### Untersuchung der Immunglobulin-bindenden Eigenschaften von IPSE

Zur Untersuchung der Immunglobulin-bindenden Eigenschaften der erfindungsgemäßen Polypeptide, insbesondere IPSE, wurden Western- und Dotblotting-Techniken eingesetzt.

Dottblotting: Polyklonales IgE (BA1004; DPC Biermann, Bad Nauheim, Deutschland) wurde in verschiedenen Mengen (1000-30 ng/Dot) auf Nitrozellulosemembran aufgetragen. Anschließend wurden freie Bindungsstellen der Membran durch Inkubation in 0,1 M Tris-gepufferter Kochsalzlösung, pH 7,4, unter Zusatz von 0,05 % (v/v) Tween 20 (Tris-Tween) blockiert und die Membran mit biotinyliertem rekombinatem IPSE (His Tag Fusionsprotein; 1 µg/ml) über Nacht bei RT inkubiert. Nach Waschen wurden die Blots mit Streptavidin-Alkalischer Phosphatase (1:5000) für 2 h inkubiert. Die Entwicklung der Blots erfolgte nach erneutem Waschen durch Zugabe des Substrat-Chromogen-Gemisches (Nitro-Blue-Tetrazolium-Bromo-Chloro-Indolyl-Phosphat).

Parallel wurden verschiedene Mengen an His Tag IPSE Fusionsprotein (1000-30 ng/Dot) auf Nitrozellulosemembran aufgetragen.

Nach Blockierung freier Bindungsstellen (siehe vorhergehender Absatz) wurde die Membran mit biotinyliertem polyklonalem IgE (1 µg/ml) über Nacht bei RT inkubiert. Die weitere Entwicklung erfolgte über Streptavidin-Alkalische Phosphatase wie im vorhergehenden Absatz.

Westernblotting: Hierzu wurden natürliches IPSE oder rekombinantes His Tag IPSE Fusionsprotein mittels SDS-PAGE (12 % T, 4 % C) in einem Standardverfahren (Lämmli, U.K., Nature 227 (1970) 680-685) unter nichtreduzierenden Bedingungen aufgetrennt und mittels Semidry-Blotting (30 min., 0,8 mA/cm) auf Nitrozellulosemembran (Schleicher & Schüll, Dassel, Deutschland) transferiert. Freie Protein-Bindungsstellen wurden durch Inkubation der Membran in Tris-Tween blockiert. Anschließend wurden Streifen der Membran über Nacht bei RT mit Seren gesunder, nichtsensibilisierter Blutspender (1:20 bzw. 1:100 in Tris-Tween) inkubiert. Nach Waschen wurden die Streifen mit Alkalische-Phosphatase-markiertem anti-human-IgE (1:2000 in Tris-Tween; Allergopharma, Reinbek, Deutschland) bzw. anti-human-IgG (1:40 000; Dianova, Hamburg, Deutschland) für 2 h bei RT inkubiert. Die Entwicklung der Blots erfolgte nach erneutem Waschen durch Zugabe des Substrat-Chromogen-Gemisches (Nitro-Blue-Tetrazolium-Bromo-Chloro-Indolyl-Phosphat).

Um die Affinität der IPSE-Bindung an IgE bzw. IgG miteinander zu vergleichen, wurden Western-Blots von His Tag IPSE (0,5 µg/cm) mit humanem IgE (100-100.000 ng/ml) inkubiert. Der Nachweis der IgE-Bindung an His Tag IPSE erfolgte durch Inkubation mit Alkalische Phosphatase-markiertem anti-human IgE (1:2000; Allergopharma, Reinbek, Deutschland) wie oben.

Es wurde festgestellt, daß IPSE die menschlichen IgG-Subklassen 1 bis 4 und, nach Papainverdau, Fc- und Fab-Fragmente sowie - mit etwa 100-fach höherer Affinität als an IgG - an IgE bindet.

## Patentansprüche

1. Protein mit der in SEQ ID NO: 2 gezeigten Aminosäuresequenz.

2. Protein nach Anspruch 1, **dadurch gekennzeichnet, dass** es glykosyliert ist.

3. Protein oder Polypeptid, **dadurch gekennzeichnet, dass** es ein Homologes, ein Derivat oder ein Fragment des Proteins nach Anspruch 1 oder 2 ist, das die Aktivität aufweist, bei humanen basophilen Granulozyten die Produktion von IL-4 und/oder IL-13 zu stimulieren.

4. Nukleinsäuremolekül, **dadurch gekennzeichnet, dass** es eine für ein Protein oder Polypeptid nach den Ansprüchen 1 bis 3 kodierende Nukleinsäuresequenz aufweist, wobei es sich nicht um ein Molekül mit folgender Sequenz handelt:

5. Nukleinsäuremolekül nach Anspruch 4 mit der in SEQ ID NO: 1 gezeigten Nukleinsäuresequenz.

6. Expressionsvektor, **dadurch gekennzeichnet, dass** er ein Nukleinsäuremolekül nach Anspruch 4 oder 5 eingebaut enthält.

7. Antikörper, **dadurch gekennzeichnet, dass** er spezifisch gegen ein Protein und/oder Polypeptid nach den Ansprüchen 1 bis 3 gerichtet ist.

8. Antikörper nach Anspruch 7, **dadurch gekennzeichnet, dass** es ein monoklonaler Antikörper ist.

9. Pharmazeutische Zusammensetzung, **dadurch gekennzeichnet, dass** sie ein Protein oder Polypeptid nach des Ansprüchen 1 bis 3 zusammen mit pharmazeutisch annehmbaren Hilfs- und Trägerstoffen enthält.

10. Protein oder Polypeptid nach den Ansprüchen 1 bis 3 zur Verwendung als Arzneimittel.

11. Verwendung eines Proteins oder Polypeptids nach den Ansprüchen 1 bis 3 zur Herstellung einer pharmazeutischen Zusammensetzung zur Behandlung von Erkrankungen, die aus der Gruppe bestehend aus Multipler Sklerose, autoimmuner Uveitis, Diabetes mellitus, rheumatoider Arthritis, Behcets Syndrom, Helicobacter pylori-Infektion, entzündlichen Darmerkrankungen, akuter Organtransplantat-Abstoßungsreaktion, spontanen rekurrenten Aborten, Nematoden-Infektionen, Schistosomiasis, allergischen Reaktionen, allergischer Rhinokonjunktivitis und allergischem Asthma bronchiale ausgewählt sind.

12. Verwendung nach Anspruch 11, **dadurch gekennzeichnet, dass** die entzündliche Darmerkrankung Morbus Crohn ist.

13. Verwendung eines Proteins oder Polypeptids nach den Ansprüchen 1 bis 3 zur Induktion oder Verstärkung der IL-4-, IL-13- und/oder IgE-Produktion in vitro, **dadurch gekennzeichnet, dass** man Zellen aus der Gruppe bestehend aus humanen basophilen Granulozyten, B-Zellen T-Zellen, NK-Zellen, Endothelzellen und Fibroblasten und anderen Zellen des Immunsystems oder anderer Gewebe sowie daraus abgeleiteten Zelllinien mit einem Protein oder Polypeptid nach den Ansprüchen 1 bis 3 inkubiert.

14. Verwendung eines Proteins oder Polypeptids nach den Ansprüchen 1 bis 3 zum Nachweis und zur Aufreinigung von Immunglobulinen oder Fragmenten derselben.

15. Verwendung nach Anspruch 14, **dadurch gekennzeichnet, dass** die Immunglobuline IgE und IgG und die Fragmente Fc und/oder Fab-Fragmente sind.

16. Verwendung nach Anspruch 14 oder 15 im Rahmen eines ELISA.

17. Verwendung nach Anspruch 14 oder 15 im Rahmen einer Affinitätschromatographie.

18. Verwendung eines Nukleinsäuremoleküls nach den Ansprüchen 4 oder 5 zur in vitro-Expression der Proteine oder Polypeptide nach einem der Ansprüche 1 bis 3.

19. Verwendung nach Anspruch 18, **dadurch gekennzeichnet, dass** man das Nukleinsäuremolekül in einen geeigneten Expressionsvektor einbaut und die Proteine oder Polypeptide rekombinant in geeigneten Wirtszellen überexprimiert.

20. Verwendung nach Anspruch 19, **dadurch gekennzeichnet, dass** die Wirtszellen Escherichia coli-, Baculovirus oder eukaryontische Zellen sind.

21. Pharmazeutische Zusammensetzung, umfassend einen Expressionsvektor nach Anspruch 6 zur Expression der kodierten Proteine oder Polypeptide in vivo.

22. Pharmazeutische Zusammensetzung, **dadurch gekennzeichnet, dass** sie einen Gentransfervektor, in den ein Nukleinsäuremolekül nach den Ansprüchen 4 oder 5 eingebaut ist, zusammen mit pharmazeutisch annehmbaren Hilfs- und/oder Trägerstoffen enthält.

23. Verfahren zur Herstellung von IL-4 und/oder IL-13 in vitro, **dadurch gekennzeichnet, dass** man Zellen aus der Gruppe bestehend aus humanen basophilen Granulozyten, B-Zellen, T-Zellen, NK-Zellen, Endothelzellen und Fibroblasten und anderen Zellen des Immunsystems oder anderer Gewebe sowie daraus abgeleiteten Zelllinien mit einem Protein oder Polypeptid nach den Ansprüchen1 bis 3 inkubiert und man IL-4 und/oder IL-13 aus dem Kulturüberstand isoliert.

24. Verfahren zur Isolierung von IL-4 induzierenden Proteinen aus parasitären Würmern, **dadurch gekennzeichnet, dass** man Ei-Antigen (EA) oder Extrakt aus Th2-bzw. IgE induzierenden Stadien parasitärer Würmer (EX) gegen Wasser dialysiert und lyophilisiert, man das lyophilisierte EA oder EX mittels Kationenaustauschchromatographie auftrennt, man die Fraktionen, die bei Inkubation humaner basophiler Granulozyten eine IL-4-Induktion bewirken, poolt, einengt, man eine Affinitätschromatographie mit NHS-aktivierter Sepharose durchführt, an die Aleuria aurantia-Agglutinin oder monoklonale Antikörper gegen ein Protein nach den Ansprüchen 1 bis 3 gekoppelt sind, man die Fraktionen, die bei Inkubation humaner basophiler Granulozyten eine IL-4 Induktion bewirken, poolt und einengt.

25. Verfahren nach Anspruch 24, **dadurch gekennzeichnet, dass** die parasitären Würmer aus der Gruppe bestehend aus Schistosoma mansoni, Schistosoma japonicum, Schistosoma haematobium, Fasciola hepatica, Dicrocoelium lanceolatum, Echinococcus multilocularis, Ascaris lumbricoides, Ascaris suum, Ankylostomaduodenale, Necator americanus, Trichuristrichiura, Brugia malayi, Onchocerca volvulus, Wuchereria bancrofti, Taenia solium, Taenia bovis und verwandten Arten ausgewählt sind.

26. Verfahren zur Isolierung des Proteins nach Anspruch 1, **dadurch gekennzeichnet, dass** man Schistosoma mansoni Ei-Antigen(SmEA) gegen Wasser dialysiert und lyophilisiert, man das lyophilisierte SmEA mittels Kationenaustauschchromatographie auftrennt, man die Fraktionen, die bei Inkubation humaner basophiler Granulozyten eine IL-4 Induktion bewirken, poolt, einengt, man eine Affinitätschromatographie mit NHS-aktivierter Sepharose durchführt, an die Aleuria aurantia-Agglutinin oder monoklonale Antikörper gegen ein Protein nach den Ansprüchen1 bis 3 gekoppelt sind, man die Fraktionen, die bei Inkubation humaner basophiler Granulozyten eine IL-4-Induktion bewirken, poolt und einengt.

27. Verfahren nach den Ansprüchen 24 bis 25, **dadurch gekennzeichnet, dass** man das erhaltene Protein isoliert.

28. Verfahren nach Anspruch 27, **dadurch gekennzeichnet, dass** man das isolierte Protein anschließend lyophilisiert.

## Claims

1. Protein having the amino acid sequence shown in SEQ ID NO: 2.

2. Protein according to claim 1, **characterized in that** it is glycosylated.

3. Protein or polypeptide, **characterized in that** it is homologue, a derivative or a fragment of the protein according to claim 1 or 2, having the activity of stimulating production of IL-4 and/or I1-13 in human basophilic granulocytes.

4. Nucleic acid molecule **characterized in that** it comprises a nucleic acid sequence encoding a protein or polypeptide according to claims 1 to 3, which is not a molecule with the following sequence:

5. Nucleic acid molecule according to claim 4, having the nucleic acid sequence shown in SEQ IS NO: 1.

6. Expression vector **characterized in that** it comprises an integrated nucleic acid molecule according to claim 4 or 5.

7. Antibody **characterized in that** it is specifically directed to a protein and/or polypeptide according to claims 1 to 3.

8. Antibody according to claim 7, **characterized in that** it is a monoclonal antibody.

9. Pharmaceutical composition **characterized in that** it comprises a protein or polypeptide according to claims 1 to 3 in combination with pharmaceutically acceptable auxiliary and carrier substances.

10. Protein or polypeptide according to claims 1 to 3 for use as a medicament.

11. Use of a protein or polypeptide according to claims 1 to 3 for the preparation of a pharmaceutical composition for treatment of diseases selected from the group consisting of multiple sclerosis, autoimmune uveitis, diabetes mellitus, rheumatoid arthritis, Behcets' syndrome, helicobacter pylori infection, inflammatory intestinal diseases, acute organ transplant rejection reaction, spontaneous recurrent abortions, infections with nematodes, schistosomiasis, allergic reactions, allergic rhinoconjunctivitis and allergic bronchial asthma.

12. Use according to claim 11, **characterized in that** the inflammatory intestinal disease is Morbus Crohn.

13. Use of a protein or polypeptide according to claims 1 to 3 for inducing or enhancing the production of Il-4, IL-13 and/or IgE in vitro, **characterized in that** cells from the group consisting of human basophilic granulocytes, B-cells, T-cells, NK-cells, endothelial cells and fibroblasts and other cells of the immune system or other tissues as well as cell lines derived therefrom are incubated with a protein or polypeptide according to claims 1 to 3.

14. Use of a protein or polypeptide according to claims 1 to 3 for detection and for purification of immunoglobulins or fragments thereof.

15. Use according to claim 14, **characterized in that** the immunoglobulins are IgE and IgG and the fragments are Fc and/or Fab-fragments.

16. Use according to claim 14 or 15 in the context of an ELISA.

17. Use according to claim 14 or 15 in the context of an affinity chromatography.

18. Use of a nucleic acid molecule according to claims 4 or 5 for in vitro expression of the proteins or polypeptides according to any of claims 1 to 3.

19. Use according to claim 18, **characterized in that** the nucleic acid molecule is integrated in a suitable expression vector and the proteins or polypeptides are recombinantly overexpressed in suitable host cells.

20. Use according to claim 19, **characterized in that** the host cells are escherichia coli, baculo virus or eukaryotic cells.

21. Pharmaceutical composition comprising an expression vector according to claim 6 for expression of the encoded proteins or polypeptides in vivo.

22. Pharmaceutical composition **characterized in that** it comprises a gene transfer vector having a nucleic acid molecule according to claims 4 or 5 integrated therein, in combination with pharmaceutically acceptable auxiliary and/or carrier substances.

23. Method for the preparation of IL-4 and/or IL-13 in vitro **characterized in that** cells from the group consisting of human basophilic granulocytes, B-cells, T-cells, NK-calls, endothelial cells and fibroblasts and other cells of the immune system or other tissues as well as cell lines derived therefrom are incubated with a protein or polypeptide according to claims 1 to 3, and IL-4 and/or IL-13 is isolated from the culture supernatant.

24. Method for the isolation of proteins, which induce IL-4, from parasitic worms, **characterized in that** egg antigen (EA) or extract from Th2 or IgE inducing stages of parasitic worms (EX) are dialyzed against water and lyophilized, the lyophilized EA or EX is separated by kation exchange chromatography, the fractions effecting an induction of IL-4 upon incubation with human basophilic granulocytes are pooled, concentrated, an affinity chromatography is carried out with NHS activated sepharose, to which aleuria aurantia agglutinin or monoclonal antibodies against a protein according to claims 1 to 3 are linked, the fractions effecting an induction of IL-4 upon incubation with human basophilic granulocytes are pooled and concentrated.

25. Method according to claim 24 **characterized in that** the parasitic worms are selected from the group consisting of Schistosoma mansoni, Schistosoma japonicum, Schistosoma haematobium, Fasciola hepatica, Dicrocoelium lanceolatum, Echinococcus multilocularis, Ascaris lumbricoides, Ascaris suum, Ankylostomaduodenale, Necator americanus, Trichuristrichiura, Brugia malayi, Onchocerca volvulus, Wuchereria bancrofti, Taenia solium, Taenia bovis and related species.

26. Method for isolating the protein according to claim 1 **characterized in that** schistosoma mansoni egg antigen (SmEA) is dialyzed against water and lyophilized, the lyophilized SmEA is separated by kation exchange chromatography, the fractions effecting an induction of IL-4 upon incubation with human basophilic granulocytes are pooled, concentrated, an affinity chromatography is carried out with NHS-activated sepharose to which aleuria aurantia agglutinin or monoclonal antibodies against a protein according to claims 1 to 3 are linked, the fractions effecting an induction of IL-4 upon incubation with human basophilic granulocytes are pooled and concentrated.

27. Method according to claims 24 to 25, **characterized in that** the obtained protein is isolated.

28. Method according to claim 27, **characterized in that** the isolated protein is then lyophilized.

## Revendications

1. Protéine ayant la séquence d'acides aminés présentée dans SEQ ID n° 2.

2. Protéine selon la revendication 1, **caractérisée en ce qu'**elle est glycosylée.

3. Protéine ou polypeptide, **caractérisé(e) en ce qu**'il/elle est un homologue, un dérivé ou un fragment de la protéine selon la revendication 1 ou 2, qui présente l'activité de stimuler la production d'IL-4 et/ou IL-5 dans des granulocytes basophiles humains.

4. Molécule d'acide nucléique, **caractérisée en ce qu'**elle présente une séquence d'acide nucléique codant une protéine ou un polypeptide selon les revendications 1 à 3, et ne consiste pas en une molécule ayant la séquence suivante :

5. Molécule d'acide nucléique selon la revendication 4, ayant la séquence d'acide nucléique présentée dans SEQ ID n° 1.

6. Vecteur d'expression, **caractérisé en ce qu'**il contient une molécule d'acide nucléique incorporée selon la revendication 4 ou 5.

7. Anticorps, **caractérisé en ce qu'**il est dirigé spécifiquement contre une protéine et/ou un polypeptide selon les revendications 1 à 3.

8. Anticorps selon la revendication 7, **caractérisé en ce qu'**il est un anticorps monoclonal.

9. Composition pharmaceutique, **caractérisée en ce qu'**elle contient une protéine ou un polypeptide selon les revendications 1 à 3, conjointement avec des adjuvants ou véhicules pharmaceutiquement acceptables.

10. Protéine ou polypeptide selon les revendications 1 à 3, pour utilisation en tant que médicament.

11. Utilisation d'une protéine ou d'un polypeptide selon les revendications 1 à 3, pour la fabrication d'une composition pharmaceutique destinée au traitement de maladies qui sont choisies dans le groupe constitué par la sclérose en plaques, l'uvéite auto-immune, le diabète sucré, la polyarthrite rhumatoïde, le syndrome de Behçet, l'infection à *Helicobacter pylori*, des maladies intestinales inflammatoires, des réactions aiguës de rejet de greffe, des avortements récurrents spontanés, des infections à nématodes, la schistosomiase, des réactions allergiques, la rhinoconjonctivite allergique et l'asthme bronchique allergique.

12. Utilisation selon la revendication 11, **caractérisé en ce que** la maladie intestinale inflammatoire est la maladie de Crohn.

13. Utilisation d'une protéine ou d'un polypeptide selon les revendications 1 à 3, pour l'induction ou l'accentuation de la production d'IL-4, d'IL-13 et/ou d'IgE in vitro, **caractérisée en ce qu'**on met des cellules choisies dans le groupe constitué par des granulocytes basophiles humains, des lymphocytes B, des lymphocytes T, des cellules NK, des cellules endothéliales et des fibroblastes et d'autres cellules du système immunitaire ou d'autres tissus, ainsi que des lignées cellulaires dérivées de ceux-ci, à incuber avec une protéine ou un polypeptide selon les revendications 1 à 3.

14. Utilisation d'une protéine ou d'un polypeptide selon les revendications 1 à 3, pour la détection et pour la purification d'immunoglobulines ou de fragments de celles-ci.

15. Utilisation selon la revendication 14, **caractérisée en ce que** les immunoglobulines sont des IgE et des IgG et les fragments sont des fragments Fc et/ou Fab.

16. Utilisation selon la revendication 14 ou 15 dans le cadre d'un dosage ELISA.

17. Utilisation selon la revendication 14 ou 15 dans le cadre d'une chromatographie d'affinité.

18. Utilisation d'une molécule d'acide nucléique selon la revendication 4 ou 5, pour l'expression in vitro des protéines ou des polypeptides selon l'une quelconque des revendications 1 à 3.

19. Utilisation selon la revendication 18, **caractérisée en ce qu'**on incorpore la molécule d'acide nucléique dans un vecteur d'expression approprié et que les protéines ou polypeptides sont surexprimées dans des cellules hôtes appropriées.

20. Utilisation selon la revendication 19, **caractérisée en ce que** les cellules hôtes sont des cellules d'*Escherichia coli*, des baculovirus ou des cellules eucaryotes.

21. Composition pharmaceutique comprenant un vecteur d'expression selon la revendication 6, pour l'expression des protéines codées ou des polypeptides codés in vivo.

22. Composition pharmaceutique, **caractérisée en ce qu'**elle contient un vecteur de transfert de gène, dans lequel est incorporée une molécule d'acide nucléique selon la revendication 4 ou 5, conjointement avec des adjuvants et/ou véhicules pharmaceutiquement acceptables.

23. Procédé pour la fabrication d'IL-4 et/ou Il-13 in vitro, **caractérisé en ce qu'**on met des cellules choisies dans le groupe constitué par des granulocytes basophiles humains, des lymphocytes B, des lymphocytes T, des cellules NK, des cellules endothéliales et des fibroblastes et d'autres cellules du système immunitaire ou d'autres tissus, ainsi que des lignées cellulaires dérivées de ceux-ci, à incuber avec une protéine ou un polypeptide selon les revendications 1 à 3 et on isole IL-4 et/ou IL-3 à partir du surnageant de culture.

24. Procédé pour l'isolement de protéines induisant IL-4 à partir de vers parasites, **caractérisé en ce qu'**on dialyse contre de l'eau et lyophilise de l'antigène Ei (EA) ou un extrait provenant de stades induisant Th2 ou IgE de vers parasites (EX), on fractionne par chromatographie d'échange de cations l'EA ou l'EX lyophilisé, on réunit les fractions qui provoquent une induction d'IL-4 lors de l'incubation de granulocytes basophiles humains, on concentre, on effectue une chromatographie d'affinité avec de la Sepharose activée par NHS, à laquelle sont couplés des anticorps monoclonaux dirigés contre une protéine selon les revendications 1 à 3 ou de l'agglutinine d'*Aleuria aurantia*, on réunit et on concentre les fractions qui provoquent une induction d'IL-4 lors de l'incubation de granulocytes basophiles humains.

25. Procédé selon la revendication 24, **caractérisé en ce que** les vers parasites sont choisis dans le groupe constitué par *Schistosoma mansoni, Schistosoma japonicum, Schistosoma haematobium, Fasciola hepatica, Dicrocoelium lanceolatum, Echinococcus multilocularis, Ascaris lumbricoides, Ascaris suum, Ankylostomaduodenale, Necator americanus, Trichuristrichiura, Brugia malayi, Onchocerca volvulus, Wuchereria bancrofti, Taenia solium, Taenia bovis* et des espèces apparentées.

26. Procédé pour l'isolement de la protéine selon la revendication 1, **caractérisé en ce qu'**on dialyse contre de l'eau et lyophilise de l'antigène Ei de *Schistosoma mansoni* (SmEA), on fractionne par chromatographie d'échange de cations le SmEA lyophilisé, on réunit les fractions qui provoquent une induction d'IL-4 lors de l'incubation de granulocytes basophiles humains, on concentre, on effectue une chromatographie d'affinité avec de la Sepharose activée par NHS, à laquelle sont couplés des anticorps monoclonaux dirigés contre une protéine selon les revendications 1 à 3 ou de l'agglutinine d'*Aleuria aurantia*, on réunit et on concentre les fractions qui provoquent une induction d'IL-4 lors de l'incubation de granulocytes basophiles humains.

27. Procédé selon les revendications 24 et 25, **caractérisé en ce qu'**on isole la protéine obtenue.

28. Procédé selon la revendication 27, **caractérisé en ce que** la protéine isolée est ensuite lyophilisée.
